# EUROPÄISCHE PATENTSCHRIFT

(11) **EP 1 846 353 B1**
(45) Veröffentlichungstag und Bekanntmachung des Hinweises auf die Patenterteilung: **23.03.2011**
(21) Anmeldenummer: 06707886.5
(22) Anmeldetag: 30.01.2006
(51) Int. Cl.: C07C 51/36, C07D 307/60, B01J 19/00, C07C 55/10

(54) **VERFAHREN ZUR STEUERUNG EINER HYDRIERUNG**
METHOD FOR CONTROLLING HYDROGENATION
PROCEDE POUR REALISER UNE HYDROGENATION

(30) Priorität: 01.02.2005 DE 102005004604
(43) Veröffentlichungstag der Anmeldung: 24.10.2007
(73) Patentinhaber: BASF SE, 67056 Ludwigshafen (DE)
(72) Erfinder: WECK, Alexander, 67251 Freinsheim (DE); RÖSCH, Markus, 55276 Dienheim (DE); WINDECKER, Gunther, 67067 Ludwigshafen (DE); HEYDRICH, Gunnar, 67117 Limburgerhof (DE); PINKOS, Rolf, 67098 Bad Dürkheim (DE); SCHUBERT, Olga, 67063 Ludwigshafen (DE); HARTH, Klaus, Taim Tam, Hong Kong (CN)
(86) Internationale Anmeldenummer: PCT/EP2006/050507
(87) Internationale Veröffentlichungsnummer: WO 2006/082165

(56) Entgegenhaltungen:
- US-A- 3 798 002
- US-A- 3 809 621

## Beschreibung

Die Erfindung betrifft ein Verfahren zur Steuerung einer Hydrierung eines Eduktes in einem Hydrierreaktor.

Im Allgemeinen werden Hydrierungen bei einem Wasserstoff-Partialdruck von 5 bis 400 bar durchgeführt. Der während der Hydrierung verbrauchte Wasserstoff muss permanent ergänzt werden. Dies erfolgt durch Zuführung von Wasserstoff aus einem Reservoir über ein Regelorgan. Dabei liegt der Druck im Reservoir höher als der Systemdruck. Das Regelorgan ist im Allgemeinen ein Regelventil.

Zur Steuerung der Menge an Wasserstoff, die dem Hydrierungsprozess zugeführt werden muss, werden derzeit zwei unterschiedliche Verfahren eingesetzt. Bei dem einen Verfahren wird der Druck im System gemessen und über einen Regler, der direkt oder mittelbar - über eine Kaskaden-Regelung - auf das Zuführorgan des Wasserstoffes wirkt, konstant gehalten. Hierbei spricht man von einer versorgungsseitigen Druckregelung. Bei dem zweiten Verfahren zur Steuerung der Ergänzungsmenge an Wasserstoff wird dem System eine fest eingestellte Menge an Wasserstoff zugeführt und der Systemdruck über ein Regler, der auf das Abgasventil des Gaskreislaufes wirkt, konstant gehalten. Hierbei spricht man von einer abgasseitigen Druckregelung.

Nachteil bei der aus dem Stand der Technik bekannten Steuerung ist es, dass Wirkungen auf den Prozess und Rückwirkungen vom Prozess nur ungenügend berücksichtigt werden. Insbesondere werden Auswirkungen des Druckes auf das Hydrierergebnis nicht berücksichtigt. So kann zum Beispiel eine Druckerhöhung abhängig von der durchzuführenden Hydrierung zu einer Überhydrierung oder Unterhydrierung führen. Überhydrierung bedeutet dabei, dass das durch die Hydrierung hergestellte gewünschte Produkt im Reaktor mit Wasserstoff reagiert und so ein nicht erwünschtes Nebenprodukt gebildet wird. Unterhydrierung bedeutet, dass bei gleicher Eduktmenge weniger Produkt erzeugt wird.

Bei den derzeit eingesetzten Verfahren zur Steuerung der Hydrierung bedeutet dies, dass zum Beispiel bei einer Temperaturerhöhung im Hydrierreaktor unter einer dadurch zunehmenden Überhydrierungsrate der Sollwert der Druckregelung manuell verändert werden muss, um den Einfluss der Temperaturerhöhung zu kompensieren.

Aus US 3 809 621 und US 3 798 002 geht ein computergestenertes System zur Raffinierung und Hydrierung von ungesättigten Kohlenwasserstoffen hervor.

Aufgabe der Erfindung ist es, ein Verfahren zur Steuerung einer Hydrierung bereitzustellen, welches keine manuelle Einstellung von Prozessparametern erfordert.

Gelöst wird die Aufgabe durch ein Verfahren zur Steuerung einer Hydrierung eines Eduktes in einem Hydrierreaktor mit folgenden Schritten:
a) Bestimmung der bei der Hydrierung umgesetzten Menge an Wasserstoff,
b) Bestimmung des Verhältnisses der umgesetzten Menge an Wasserstoff zur zugeführten Menge an Edukt,
c) Vergleich dessen Schritt b) gebildeten Quotienten mit einem vorgegebenen Sollwert und
d) Änderung mindestens eines Prozessparameters, wenn der in Schritt b) gebildete Quotient vom vorgegebenen Sollwert um einen vorgegebenen Wert abweicht.

Durch die Bestimmung des Verhältnisses der umgesetzten Menge an Wasserstoff zur zugeführten Menge an Edukt wird eine gleichbleibende Produktqualität erhalten, da hierdurch gewährleisten wird, dass immer eine annähernd gleiche Menge an Wasserstoff mit einer bestimmten Menge an Edukt reagiert. Auf diese Weise wird eine Über- oder Unterhydrierung vermieden.

Der Prozessparameter in Schritt d), der geändert wird, wenn der Quotient aus der umgesetzten Menge an Wasserstoff zur zugeführten Menge an Edukt von einem vorgegebenen Sollwert um einen vorgegebenen Wert abweicht, ist zum Beispiel die Temperatur oder der Druck im Hydrierreaktor oder die Menge an zugeführtem Wasserstoff.

Der Sollwert, mit dem der Quotient aus der umgesetzten Menge an Wasserstoff und der zugeführten Menge an Edukt verglichen wird, ist dabei abhängig vom Druck und der Temperatur im Hydrierreaktor sowie dem gewünschten Hydrierprodukt.

Die bei der Hydrierung umgesetzte Menge an Wasserstoff wird vorzugsweise durch die Differenz der aus dem Prozess abgeführten Menge an Wasserstoff und der dem Prozess zugeführten Menge an Wasserstoff gebildet. Um die dem Prozess zugeführte Menge an Wasserstoff zu bestimmen, ist vorzugsweise an der Zuführungsleitung zum Hydrierreaktor, über die der Wasserstoff zugeführt wird, ein Durchflussmesser angeordnet. Zur Durchflussmessung eignen sich volumetrische Verfahren, Wirkdruckverfahren, induktive Verfahren und Ultraschall-Verfahren, Drucksonden sowie Thermosonden. Bei volumetrischen Verfahren zur Durchflussmessung werden zum Beispiel Volumenzähler wie Ovalradzähler oder Turbinenzähler eingesetzt, die Durchflussmessung mittels Wirkdruckverfahren erfolgt über Blenden, Düsen oder Venturi-Rohre, Drucksonden sind zum Beispiel das Pitotrohr oder das Prandtlstaurohr. Geeignete Thermosonden sind zum Beispiel Hitzdrahtanemometer. Die Durchflussmessung kann jedoch auch mit jedem anderen, dem Fachmann bekannten Verfahren durchgeführt werden.

Die aus dem Prozess abgeführte Menge an Wasserstoff wird vorzugsweise in einer Abgasleitung gemessen, über die nicht umgesetzte Reaktionsgase abgeführt werden. Die Bestimmung der Menge an Wasserstoff erfolgt auch hier vorzugsweise mit einem Durchflussmesser. Als Messverfahren eignen sich die gleichen Verfahren wie auch bei der Durchflussmessung zur Bestimmung der zugeführten Menge an Wasserstoff.

Die über die Abgasleitung abgeführten Gase werden vorzugsweise als Kreisgas erneut dem Prozess zugeführt.

In einer bevorzugten Ausführungsform ist dem Hydrierreaktor ein Produktabscheider nachgeschaltet, in welchem das Hydrierprodukt von nicht umgesetzten Reaktionsgasen abgetrennt wird. Aus dem Produktabscheider führt die Abgasleitung, über welche die nicht umgesetzten Reaktionsgase abgeführt werden. Zur Trennung der Hydrierprodukte von den nicht umgesetzten Reaktionsgasen wird das den Hydrierreaktor verlassende Gemisch in mindestens einem Wärmetauscher abgekühlt, so dass das Hydrierprodukt auskondensiert. Im Produktabscheider erfolgt dann eine Flüssig/Gas-Phasentrennung.

In einer bevorzugten Ausführungsform wird aus dem Vergleich des aus der umgesetzten Menge an Wasserstoff zur zugeführten Menge an Edukt gebildeten Quotienten mit einem vorgegebenen Sollwert ein Sollwert für einen Temperaturregler generiert, wobei der Temperaturregler die Temperatur im Hydrierreaktor regelt. Da die Hydrierrate von der Temperatur abhängig ist, wird anhand des auf die Menge an Edukt bezogenen umgesetzten Wasserstoffes über die Temperatur im Hydrierreaktor die gewünschte Produktqualität erreicht. Eine Über- oder Unterhydrierung wird vermieden.

Der Druck im Hydrierreaktor wird wie bei den aus dem Stand der Technik bekannten Verfahren zulaufseitig oder abgasseitig geregelt. Bei der zulaufseitigen Druckregelung wird der Druck im System gemessen und über einen Regler, der direkt oder mittelbar mittels einer Kaskaden-Regelung auf das Zuführorgan des Wasserstoffes wirkt, konstant gehalten. Das Zuführorgan des Wasserstoffs ist dabei vorzugsweise ein Regelventil.

Bei der abgasseitigen Regelung wird ebenfalls der Druck im System gemessen und über einen Regler, der auf ein Abgasventil in der Abgasleitung wirkt, konstant gehalten.

In einer bevorzugten Ausführungsform wird das Verhältnis von umgesetztem Wasserstoff zu zugeführten Menge an Edukt durch Einstellung der zugeführten Menge an Wasserstoff konstant gehalten. Weiterhin wird der sich im Hydrierreaktor einstellende Druck durch Regeln der Temperatur konstant gehalten, indem der sich im Hydrierreaktor einstellende Druck in einem Regler mit einem Solldruck verglichen wird und durch Abweichung des Istwertes vom Sollwert eine Regelgröße für die Temperatur generiert wird.

Um das Verhältnis von umgesetztem Wasserstoff zu zugeführter Menge an Edukt durch Einstellung der zugeführten Menge an Wasserstoff konstant zu halten, wird von dem Regler, in welchem das Verhältnis der umgesetzten Menge an Wasserstoff zur zugeführten Menge an Edukt gebildet wird, bei Abweichung dieses Quotienten von einem vorgegebenen Sollwert der Durchflussquerschnitt in einem Regelventil, welches die Menge an zugeführtem Wasserstoff regelt, vergrößert oder verkleinert. Hierzu wird das Regelventil abhängig von einer Stellgröße, die von dem Regler, in welchem der Quotient aus der umgesetzten Menge an Wasserstoff zur zugeführten Menge an Edukt gebildet wird, an das Regelventil übertragen wird, weiter geöffnet, wenn mehr Wasserstoff benötigt wird oder weiter geschlossen, wenn weniger Wasserstoff benötigt wird. Durch die Öffnungs- oder Schließbewegung des Regelventils wird der Durchflussquerschnitt im Regelventil vergrößert oder verkleinert, so dass mehr oder weniger Wasserstoff durch dieses hindurchströmt.

Ein Konstanthalten des Druckes im Hydrierreaktor durch Regeln der Temperatur im Hydrierreaktor ist möglich, weil sich bei einem vorgegebenen Druck und einer vorgegebenen Temperatur bei einer Änderung des Druckes das Verhältnis der umgesetzten Menge an Wasserstoff zur zugeführten Menge an Edukt ändert, was zu einer Änderung der Hydrierrate und damit zu einer Temperaturänderung im Hydrierreaktor führt. Durch eine Temperaturänderung wird die vorgesehene Hydrierrate wieder eingestellt, was dazu führt, dass sich wieder das vorgegebene Verhältnis der umgesetzten Menge an Wasserstoff zur zugeführten Menge an Edukt einstellt und dieses wiederum dazu führt, dass sich der Druck im Hydrierreaktor so ändert, dass sich wieder der vorgegebene Druck einstellt.

In einer weiteren Ausführungsform wird das Verhältnis von umgesetztem Wasserstoff zu zugeführter Menge an Edukt sowie die Temperatur im Hydrierreaktor konstant gehalten. Eine Regelung des Druckes im Hydrierreaktor erfolgt nicht. Dies ist dann möglich, wenn die Abhängigkeit der Hydrieraktivität vom Systemdruck so stark ist, dass das System bei einer konstanten Temperatur nur einen möglichen Betriebspunkt besitzt. In diesem Falle ist das System bezüglich des Druckes selbstregelnd. Der gewünschte Hydriergrad stellt sich entsprechend des Verhältnisses der umgesetzten Menge an Wasserstoff zur zugeführten Menge an Edukt ein. Sobald der Verbrauch an Wasserstoff im Hydrierreaktor geringer ist als die Zufuhr, steigt der Systemdruck aufgrund der Wasserstoffakkumulation an. Hierdurch erhöht sich die Aktivität des Katalysators und zusätzlich steigt die Verweilzeit im Hydrierreaktor aufgrund der erhöhten Gasdichte an. Dies führt wiederum zu einem Anstieg der Reaktionsgeschwindigkeit. Dabei steigt die Reaktionsgeschwindigkeit solange an, bis die Menge an zugeführtem Wasserstoff wieder der Menge an umgesetztem Wasserstoff entspricht. Wenn im umgekehrten Fall die Menge an umgesetztem Wasserstoff größer ist als die Menge an zugeführtem Wasserstoff, sinkt der Systemdruck aufgrund des abnehmenden Gehaltes an Wasserstoff im Hydrierreaktor. Dies führt zu einer Verringerung der Aktivität des Katalysators und gleichzeitig zu einer Verringerung der Verweilzeit im Reaktor. Aus der Verringerung der Aktivität des Katalysators resultiert eine Abnahme der Reaktionsgeschwindigkeit. Dabei nimmt die Reaktionsgeschwindigkeit solange ab, bis die Menge an umgesetztem Wasserstoff und die Menge an zugeführtem Wasserstoff wieder gleich groß sind.

In einer bevorzugten Ausführungsform wird die Temperatur im Hydrierreaktor über die Temperatur eines Wärmeträgermediums eingestellt. Das Wärmeträgermedium strömt dabei vorzugsweise durch mindestens ein im Reaktor angeordnetes Rohr und/oder in einer als Doppelmantel ausgeführten Reaktorwand. In einer bevorzugten Ausführungsform ist der Hydrierreaktor als Rohrbündelwärmetauscher ausgebildet. Dabei werden die Rohre vom Wärmeträgermedium umströmt und das Reaktionsgemisch strömt durch die Rohre.

Abhängig von der Temperatur, bei der die Hydrierung durchgeführt wird, eignen sich als Wärmeträgermedien zum Beispiel Wasser, Wärmeträgeröle oder Salzschmelzen.

Wenn Wasser als Wärmeträgermedium eingesetzt wird, wird der Druck des Wassers vorzugsweise so gewählt, dass das Wasser durch Wärmeaufnahme von der exotherm ablaufenden Hydrierung verdampft.

In einer bevorzugten Ausführungsform wird die Hydrierung in Gegenwart eines heterogenen Katalysators durchgeführt. Als Aktivkomponente enthält der Katalysator vorzugsweise Cu, Pt, Rh, Pd oder Mischungen daraus. Der Katalysator kann zum Beispiel als Gewebe, Gestrick, regelmäßige Packung, Füllkörper oder Granulat, welche jeweils die Aktivkomponente enthält, in den Reaktor eingebracht sein. Dabei können das Gewebe, Gestrick, die geordnete Packung, die Füllkörper oder das Granulat aus der Aktivkomponente gefertigt sein, die Aktivkomponente zum Beispiel als Bestandteil einer Legierung enthalten oder mit der Aktivkomponente beschichtet sein. Auch ist es möglich, die Reaktorwand mit der Aktivkomponente zu beschichten.

Bei Verwendung eines Reaktors in Form eines Rohrbündelwärmetauschers ist der Katalysator vorzugsweise als Gewebe, Gestrick, geordnete Packung oder Füllkörperschüttung in die einzelnen Rohre des Rohrbündels eingebracht.

In einer bevorzugten Ausführungsform enthält der Hydrierreaktor mindestens eine Dampfphase und/oder mindestens eine Flüssigphase oder ein überkritisches Fluid. Vorzugsweise enthält der Hydrierreaktor mindestens eine Dampfphase, in welcher die Hydrierung erfolgt.

In einer bevorzugten Ausführungsform enthält der Eduktstrom eine oder mehrere der Verbindungen, ausgewählt aus Maleinsäure, Maleinsäureanhydrid, Maleinsäure(halb)ester und weiteren Maleinsäureanhydrid-Derivaten. Durch die Hydrierung werden aus diesen Verbindungen, die der Eduktstrom enthält, eine oder mehrere der Verbindungen, ausgewählt aus Bernsteinsäure, Bernsteinsäureanhydrid, γ-Butyrolacton, Butandiol, Tetrahydrofuran und Butanol erhalten.

Die Hydrierung wird vorzugsweise bei einem Druck im Bereich von 5 bis 100 bar, besonders bevorzugt im Bereich von 5 bis 30 bar durchgeführt. Die Temperatur der Hydrierung liegt vorzugsweise im Bereich von 170°C bis 300°C und besonders bevorzugt im Bereich von 200°C bis 280°C.

Bei der Hydrierung von Maleinsäure, Maleinsäureanhydrid, Maleinsäure(halb)ester und/oder weiteren Maleinsäureanhydrid-Derivaten wird das Verhältnis der umgesetzten Menge an Wasserstoff zur zugeführten Menge an Edukt vorzugsweise so gewählt, dass bei der Durchführung der Hydrierung bei einem Druck im Bereich von 5 bis 30 und einer Temperatur im Bereich von 200°C bis 280°C in der Gasphase, ein Produkt entsteht, welches 0 bis 80 % γ-Butyrolacton, 20 bis 100 % Tetrahydrofuran und maximal 10 % Nebenprodukte enthält. Nebenprodukte die bei dieser Hydrierung entstehen sind zum Beispiel Butanol, Bernsteinsäureanhydrid, Bernsteinsäure oder Butan.

Im Folgenden wird die Erfindung anhand einer Zeichnung näher beschrieben.

Darin zeigt:
- Figur 1: eine Steuerung einer Hydrierung in einer ersten Ausführungsform,
- Figur 2: eine Steuerung einer Hydrierung in einer zweiten Ausführungsform und
- Figur 3: eine Steuerung einer Hydrierung in einer dritten Ausführungsform.

Im Folgenden bezeichnen gleiche Bezugszeichen gleiche Bauteile.

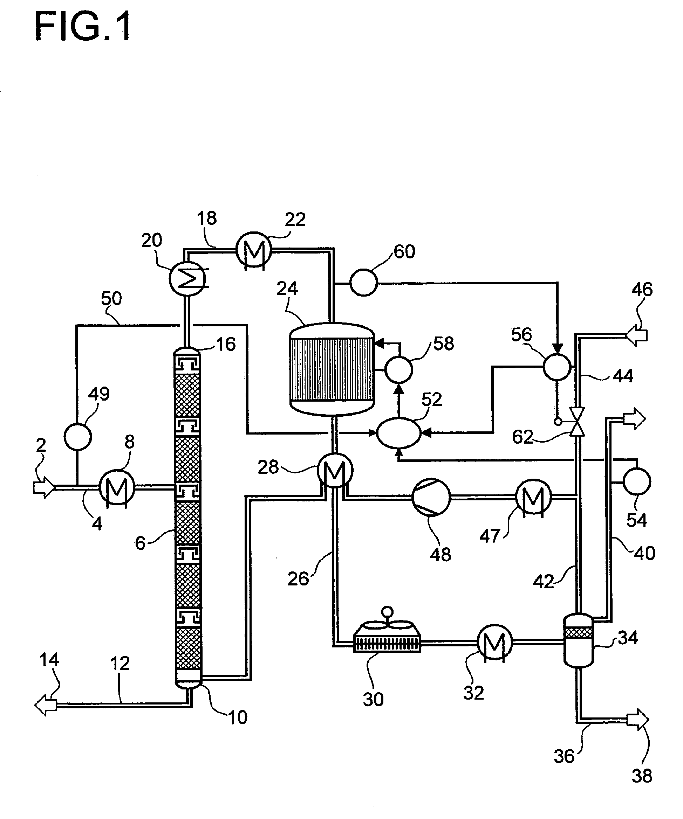

Figur 1 zeigt eine Steuerung einer Hydrierung in einer ersten Ausführungsform.

Zur Hydrierung wird an einer Eduktzufuhr 2 Edukt dem Prozess zugeführt. Hierzu ist das Edukt vorzugsweise in einem Lösemittel gelöst. Über eine Zuführleitung 4 strömt das Gemisch aus Edukt und Lösemittel in eine Kolonne 6. In der Zuführleitung 4 ist ein erster Wärmetauscher 8 aufgenommen, in welchem das Gemisch aus Edukt und Lösemittel vorgewärmt wird.

In der Kolonne 6 wird das vorgewärmte Gemisch aus Edukt und Lösemittel in Edukt und Lösemittel getrennt. Die Trennung erfolgt dabei vorzugsweise durch Destillation. Zur Trennung des Gemisches aus Edukt und Lösemittel sind in der Kolonne vorzugsweise Einbauten enthalten. Geeignete Einbauten sind zum Beispiel Böden, wie Glockenböden, Siebböden, Tunnelböden oder andere dem Fachmann bekannte Böden, oder Packungen, wie Füllkörper, Schüttungen, geordnete Packungen oder Gewirke oder Gestricke.

Am Sumpf 10 der Kolonne 6 wird vorgewärmter Wasserstoff zugeführt. Die zur Trennung von Edukt und Lösemitteln notwendige Wärme wird über den vorgewärmten Wasserstoff in die Kolonne 6 eingebracht. Der gasförmige Wasserstoff strömt vom Sumpf 10 zum Kopf 16 der Kolonne 6 und gibt dabei Wärme an das Gemisch aus Edukt und Lösemittel ab, wobei die leichter siedenden Komponenten aus diesem Gemisch verdampfen und so von den schwerer siedenden getrennt werden.

Am Sumpf 10 der Kolonne 6 wird das Lösemittel über eine Lösemittelentnahmeleitung 12 zu einer Lösemittelentnahme 14 geführt.

Das in der Kolonne 6 abgetrennte Lösemittel wird in einer bevorzugten Ausführungsform erneut zum Lösen des Edukts verwendet. Hierzu kann das Lösemittel vor dem erneuten Einsatz aufbereitet werden, wenn im Lösemittel noch Verunreinigungen enthalten sind.

Das in der Kolonne 6 abgetrennte Edukt wird zusammen mit dem Wasserstoff über den Kopf 16 der Kolonne 6 abgezogen. Edukt und Wasserstoff strömen über eine Verbindungsleitung 18 zunächst in einen Partialkondensator 20, von dort weiter in einen Vorwärmer 22 und schließlich in einen Hydrierreaktor 24.

Im Partialkondensator 20 wird der Edukt und Wasserstoff enthaltende, den Kopf 16 der Kolonne 6 verlassende Dampfstrom abgekühlt, wobei ein Teil des Dampfstromes auskondensiert. Hierdurch werden vor allem noch im Dampfstrom enthaltene Komponenten, deren Siedetemperatur oberhalb der Siedetemperatur des Eduktes liegt, auskondensiert. Die im Partialkondensator 20 auskondensierten Substanzen strömen zurück in die Kolonne 6. Der weiterhin dampfförmig vorliegende, Edukt und Wasserstoff enthaltende Strom wird dann im Vorwärmer 22 auf die zur Hydrierung erforderliche Temperatur erwärmt. Die Hydrierung erfolgt im Hydrierreaktor 24.

In der in Figur 1 dargestellten Ausführungsform ist der Hydrierreaktor in Form eines Rohrbündelwärmetauschers ausgeführt. In den Rohren des Hydrierreaktors 24 ist vorzugsweise ein heterogener Katalysator enthalten, der als Aktivkomponente Cu, Pt, Rh, Pd oder Mischungen daraus enthält. Der Katalysator liegt dabei vorzugsweise im Form eines Gewebes, Gestricks, einer geordneten Packung oder als Füllkörper vor.

Neben der hier dargestellten Ausführungsform, in der der Reaktor als Rohrbündelwärmetauscher ausgebildet, ist es auch möglich, einen Rohrreaktor, einen Wirbelschichtreaktor oder jeden anderen, dem Fachmann bekannten, für Hydrierungen geeigneten Reaktor einzusetzen.

Über eine Entnahmeleitung 26 wird ein produkthaltiger Gasstrom aus dem Hydrierreaktor 24 abgeführt. Der produkthaltige Gasstrom strömt zunächst durch einen zweiten Wärmetauscher 28, in welchem dieser Wärme an den wasserstoffhaltigen Gasstrom abgibt, der am Sumpf 10 der Kolonne 6 zugeführt wird. Hierdurch erfolgt bereits eine Abkühlung des produkthaltigen Gasstromes. In mindestens einem Produktkondensator wird zumindest das Produkt aus dem produkthaltigen Gasstrom kondensiert. Als Produktkondensatoren können zum Beispiel luftgekühlte Kondensatoren 30 oder Kondensatoren 32, die mit einem beliebigen flüssigen oder dampfförmigen Wärmeträgermedium gespeist werden, eingesetzt werden. Besonders geeignet sind flüssige Wärmeträgermedien, die durch die Wärmeaufnahme im Kondensator 32 verdampfen. Ganz besonders geeignet ist gerade siedendes Wasser unter verringertem Druck.

Die Entnahmeleitung 26, in der die Produktkondensatoren 30, 32 aufgenommen sind, mündet in einen Produktabscheider 34. Im Produktabscheider 34 wird das in flüssiger Form vorliegende Produkt von gasförmigen Bestandteilen getrennt. Das in flüssiger Form vorliegende Produkt gelangt über eine Produktentnahmeleitung 36 zu einer Produktentnahme 38.

Über eine Abgasleitung 40 am Kopf des Produktabscheiders 34 wird ein wasserstoffhaltiger Abgasstrom abgeführt. Der wasserstoffhaltige Abgasstrom wird vorzugsweise als Kreisgas zur Ergänzung des bei der Hydrierung im Hydrierreaktor 24 umgesetzten Wasserstoffes wieder eingesetzt.

Über eine zweite Leitung 42, die ebenfalls am Kopf des Produktabscheiders 34 angeordnet ist, wird ebenfalls ein wasserstoffhaltiger Gasstrom abgezogen. In die Leitung 42 mündet eine Wasserstoffleitung 44, über welche Wasserstoff von einer Wasserstoffzufuhr 46 zugeführt wird. Die Menge des über die Wasserstoffleitung 44 entspricht dabei vorzugsweise der im Hydrierreaktor 24 umgesetzten Wasserstoffmenge. In einer besonders bevorzugten Ausführungsform wird der Wasserstoff, der über die Abgasleitung 40 aus dem Prozess abgezogen wurde, nach einer optional durchgeführten Aufbereitung über die Wasserstoffleitung 44 erneut dem Prozess zugeführt.

Der über die Leitung 42 aus dem Produktabscheider 34 abgezogene wasserstoffhaltige Gasstrom und der über die Wasserstoffleitung 44 zugeführte zusätzliche Wasserstoff werden in einen dritten Wärmetauscher 47 erwärmt, in einem Verdichter 48 auf Systemdruck komprimiert und anschließend im zweiten Wärmetauscher 28 durch Wärmeaufnahme vom gasförmigen Produktstrom, der aus dem Hydrierreaktor 24 abgezogen wurde, weiter erwärmt. Der so vorgewärmte wasserstoffhaltige Gasstrom wird dann dem Sumpf 10 der Kolonne 6 zugeführt.

Zur Steuerung der Hydrierung wird mit einem ersten Durchflussmesser 49 die durch die Zuführleitung 4 strömende Menge an Edukt gemessen. Der vom ersten Durchflussmesser 49 erfasste Messwert wird über eine Datenleitung 50 an einen Regler 52 übermittelt.

Mit einem zweiten Durchflussmesser 54 wird die Menge des wasserstoffhaltigen Gases gemessen, das über die Abgasleitung 40 den Prozess verlässt. Dieser Messwert wird ebenfalls dem Regler 52 zugeführt. Mit einem Durchflussregler 56 wird die dem Prozess zugeführte Menge an Wasserstoff gemessen. Hierzu ist der Durchflussregler 56 an der Wasserstoffleitung 44 angeordnet. Der Messwert des Durchflussreglers 56 wird ebenfalls dem Regler 52 zugeführt. Im Regler 52 wird die Differenz der Messwerte des zweiten Durchflussmessers 54 und des Durchflussreglers 56 gebildet und diese ins Verhältnis mit dem Messwert des ersten Durchflussmessers 49 gesetzt. Dieser Quotient wird mit einem vorgegebenen Sollwert verglichen und liefert so einen Sollwert, der an einen Temperaturregler 58 übertragen wird. Über den Temperaturregler 58 wird die Temperatur im Hydrierreaktor 24 eingestellt. Die Temperaturreglung erfolgt dabei vorzugsweise über die Temperatur und den Volumenstrom des den Hydrierreaktor 24 durchströmenden Wärmeträgermediums.

Mit einem Druckregler 60 wird der Systemdruck in der zum Hydrierreaktor 24 führenden Verbindungsleitung 18 gemessen. Dieser Messwert wird als Stellgröße dem Durchflussregler 56 zugeführt. Der Durchflussregler 56 wirkt auf ein Regelventil 62, welches in der Wasserstoffleitung 44 angeordnet ist. Über das Regelventil 62 wird die dem Prozess zuzuführende Wasserstoffmenge eingestellt.

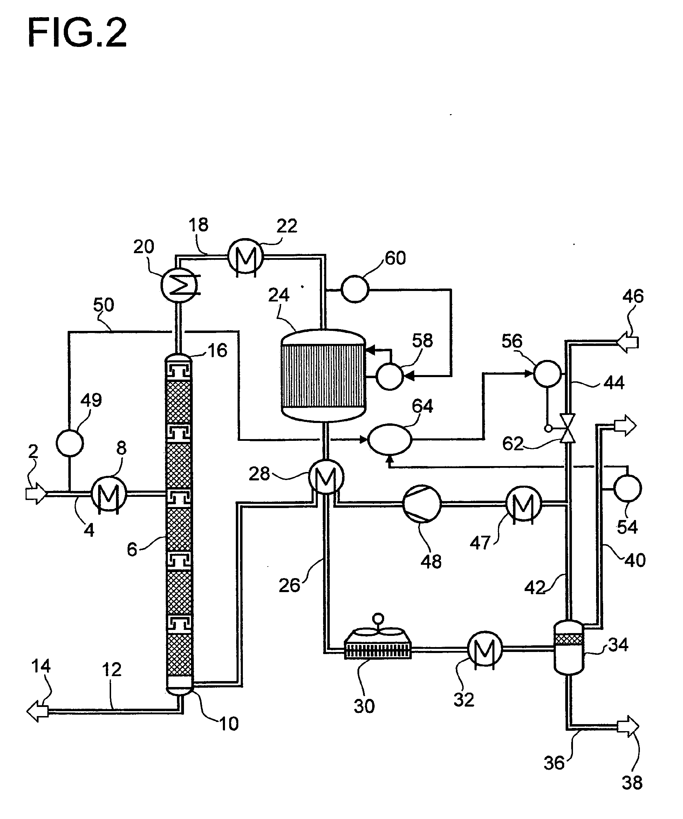

Figur 2 zeigt eine Steuerung einer Hydrierung in einer zweiten Ausführungsform.

Die in Figur 2 dargestellte Ausführungsform unterscheidet sich von der in Figur 1 dargestellten lediglich durch die Art der Steuerung der Hydrierung. Bei der in Figur 2 dargestellten Ausführungsform wird der durch den ersten Durchflussmesser 49 erfasste Messwert an einen Regler 64 übertragen. Weiterhin empfängt der Regler 64 den vom zweiten Durchflussmesser 54 erfassten Messwert.

Ziel der in Figur 2 dargestellten Regelung ist es, das Verhältnis der Menge an umgesetztem Wasserstoff zur Menge an Edukt konstant zu halten. Hierzu wird im Regler 62 aus dem Verhältnis der umgesetzten Menge an Wasserstoff zur Menge an Edukt die Menge an zuzuführendem Wasserstoff gewonnen. Dieser Wert wird als Stellgröße an den Durchflussregler 56 übertragen. Der Durchflussregler 56 gibt ein Stellsignal an das Regelventil 62, über welches die Menge an zuzuführendem Wasserstoff eingestellt wird. Auf diese Weise wird das Verhältnis der umgesetzten Menge an Wasserstoff zur zugeführten Menge an Edukt konstant gehalten.

Auch in der in Figur 2 dargestellten Ausführungsform erfasst der Druckregler 60 den Druck in der Verbindungsleitung 18 bevor diese in den Hydrierreaktor 24 mündet. Der Druckregler 60 liefert eine Stellgröße für den Temperaturregler 58, über welchen die Temperatur im Hydrierreaktor 24 eingestellt wird. Auf diese Weise wird der Betriebspunkt, bei dem das beste Hydrierergebnis erzielt wird, im Hydrierreaktor 24 eingestellt. Der Betriebspunkt ist dabei durch Druck, Temperatur und das Verhältnis der umgesetzten Menge an Wasserstoff zur zugeführten Menge an Edukt definiert.

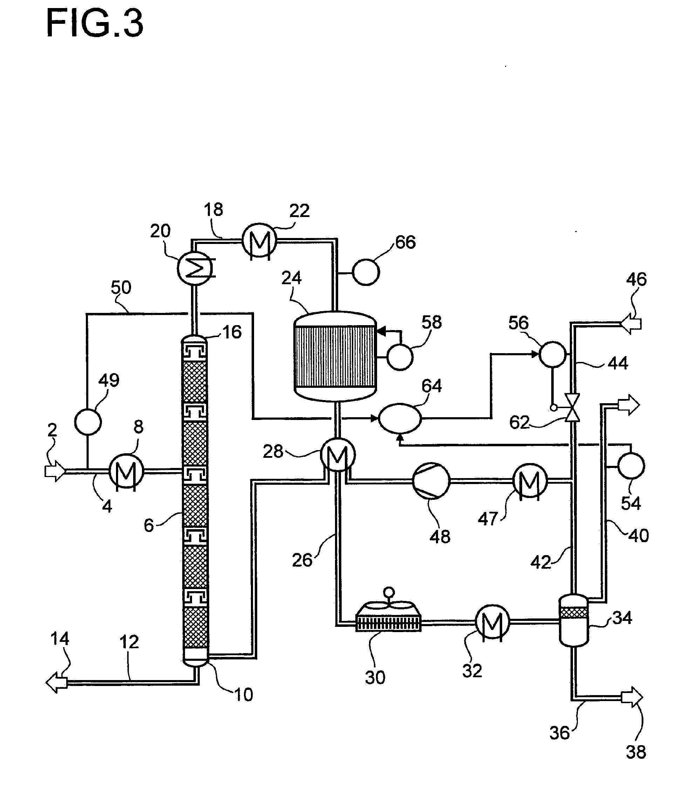

Figur 3 zeigt eine Steuerung einer Hydrierung in einer dritten Ausführungsform.

Die in Figur 3 dargestellte Ausführungsform unterscheidet sich von der in Figur 2 dargestellten dadurch, dass die Temperatur im Hydrierreaktor 24 konstant gehalten wird und sich der Druck im System selber einstellt. Hierzu ist lediglich in der Verbindungsleitung 18 ein Druckmesser 66 angeordnet, mit dem der Druck im System gemessen wird.

Die Temperatur wird konstant gehalten, indem mit dem Temperaturregler 58 die Temperatur im Hydrierreaktor 24 gemessen wird und anhand des Temperaturmesswertes die Temperatur und die Durchflussmenge des Wärmeträgermediums eingestellt wird.

Auch bei der in Figur 3 dargestellten Ausführungsform wird entsprechend der in Figur 2 dargestellten Ausführungsform das Verhältnis der umgesetzten Menge an Wasserstoff zur zugeführten Menge Edukt konstant gehalten, wozu dem Regler 64 die Messwerte des ersten Durchflussmessers 49 und des zweiten Durchflussmessers 54 zugeführt werden und die Menge an zuzuführendem Wasserstoff als Stellgröße vom Regler 64 dem Durchflussregler 56 zugeführt wird. Die Menge an zuzuführendem Wasserstoff wird über das Regelventil 62, welches vom Durchflussregler 56 eine Stellgröße erhält, eingestellt.

### Bezugszeichen

- 2: Eduktzufuhr
- 4: Zuführleitung
- 6: Kolonne
- 8: erster Wärmetauscher
- 10: Sumpf
- 12: Lösemittelentnahmeleitung
- 14: Lösemittelentnahme
- 16: Kopf
- 18: Verbindungsleitung
- 20: Partialkondensator
- 22: Vorwärmer
- 24: Hydrierreaktor
- 26: Entnahmeleitung
- 28: zweiter Wärmetauscher
- 30: luftgekühlter Kondensator
- 32: Kondensator
- 34: Produktabscheider
- 36: Produktentnahmeleitung
- 38: Produktentnahme
- 40: Abgasleitung
- 42: Leitung
- 44: Wasserstoffleitung
- 46: Wasserstoffzufuhr
- 47: Wärmetauscher
- 48: Verdichter
- 49: erster Durchflussmesser
- 50: Datenleitung
- 52: Regler
- 54: zweiter Durchflussmesser
- 56: Durchflussregler
- 58: Temperaturregler
- 60: Druckregler
- 62: Regelventil
- 64: Regler
- 66: Druckmesser

## Patentansprüche

1. Verfahren zur Steuerung einer Hydrierung eines Eduktes in einem Hydrierreaktor mit folgenden Schritten:
a) Bestimmung der bei der Hydrierung umgesetzten Menge an Wasserstoff,
b) Bestimmung des Verhältnisses der umgesetzten Menge an Wasserstoff zur zugeführten Menge an Edukt,
c) Vergleich des in Schritt b) gebildeten Quotienten mit einem vorgegebenen Sollwert und
d) Änderung mindestens eines Prozessparameters, wenn der in Schritt b) gebildete Quotient vom vorgegebenen Sollwert um einen vorgegebenen Wert abweicht.

2. Verfahren nach Anspruch 1, **dadurch gekennzeichnet, dass** zur Bestimmung der umgesetzten Menge an Wasserstoff die Differenz der aus dem Prozess abgeführten Menge und der dem Prozess zugeführten Menge an Wasserstoff gebildet wird.

3. Verfahren nach Anspruch 1 oder 2, **dadurch gekennzeichnet, dass** aus dem in Schritt c) durchgeführten Vergleich ein Sollwert für einen Temperaturregler, der die Temperatur im Hydrierreaktor regelt, generiert wird.

4. Verfahren nach Anspruch 3, **dadurch gekennzeichnet, dass** der Druck im Hydrierreaktor mittels einer zulaufseitigen oder ablaufseitigen Druckregelung konstant gehalten wird.

5. Verfahren nach Anspruch 1 oder 2, **dadurch gekennzeichnet, dass** das Verhältnis von umgesetztem Wasserstoff zu zugeführter Menge an Edukt durch Einstellung der zugeführten Menge an Wasserstoff konstant gehalten wird und der sich im Hydrierreaktor einstellende Druck durch Regeln der Temperatur konstant gehalten wird, indem der sich im Hydrierreaktor einstellende Druck in einem Regler mit einem Solldruck verglichen wird und durch die Abweichung des Istwertes vom Sollwert eine Regelgröße für die Temperatur generiert wird.

6. Verfahren nach Anspruch 1 oder 2, **dadurch gekennzeichnet, dass** das Verhältnis von umgesetztem Wasserstoff zu zugeführter Menge an Edukt sowie die Temperatur im Hydrierreaktor konstant gehalten werden.

7. Verfahren nach einem der Ansprüche 1 bis 6, **dadurch gekennzeichnet, dass** die Temperatur im Hydrierreaktor über die Temperatur eines Wärmeträgermediums eingestellt wird.

8. Verfahren nach Anspruch 7, **dadurch gekennzeichnet, dass** das Wärmeträgermedium durch mindestens ein im Hydrierreaktor angeordnetes Rohr und/oder in einer als Doppelmantel ausgeführten Reaktorwand strömt.

9. Verfahren nach einem der Ansprüche 1 bis 8, **dadurch gekennzeichnet, dass** die Hydrierung in Gegenwart eines heterogenen Katalysators durchgeführt wird.

10. Verfahren nach Anspruch 9, **dadurch gekennzeichnet, dass** der Katalysator als Aktivkomponente Cu, Pt, Rh, Pd oder Mischungen daraus enthält.

11. Verfahren nach einem der Ansprüche 1 bis 10, **dadurch gekennzeichnet, dass** der Hydrierreaktor mindestens eine Dampfphase und/oder mindestens eine Flüssigphase oder ein überkritisches Fluid enthält.

12. Verfahren nach einem der Ansprüche 1 bis 11, **dadurch gekennzeichnet, dass** der Eduktstrom eine oder mehrere der Verbindungen, ausgewählt aus Maleinsäure, Maleinsäureanhydrid, Maleinsäure(halb)ester und weiteren Maleinsäureanhydrid-Derivaten, enthält, und durch die Hydrierung eine oder mehrere der Verbindungen, ausgewählt aus Bernsteinsäure, Bernsteinsäureanhydrid, γ-Butyrolacton, Butandiol, Tetrahydrofuran und Butanol erhalten werden.

13. Verfahren nach einem der Ansprüche 1 bis 12, **dadurch gekennzeichnet, dass** die Hydrierung bei einer Temperatur im Bereich von 170 bis 300°C und bei einem Druck im Bereich von 5 bis 100 bar durchgeführt wird.

## Claims

1. A method of controlling a hydrogenation of a starting material in a hydrogenation reactor, which comprises the following steps:
a) determination of the amount of hydrogen reacted in the hydrogenation,
b) determination of the ratio of the amount of hydrogen reacted to the amount of starting material fed in,
c) comparison of the ratio derived in step b) with a prescribed value and
d) alteration of at least one process parameter if the ratio derived in step b) deviates by a prescribed amount from the prescribed value.

2. The method according to claim 1, wherein the difference between the amount of hydrogen discharged from the process and the amount of hydrogen fed into the process is derived to determine the amount of hydrogen reacted.

3. The method according to claim 1 or 2, wherein a set point for a temperature regulator which regulates the temperature in the hydrogenation reactor is generated from the comparison carried out in step c).

4. The method according to claim 3, wherein the pressure in the hydrogenation reactor is kept constant by means of a feed-side or discharge-side pressure regulation.

5. The method according to claim 1 or 2, wherein the ratio of hydrogen reacted to amount of starting material fed in is kept constant by adjusting the amount of hydrogen fed in and the pressure established in the hydrogenation reactor is kept constant by regulating the temperature by comparing the pressure established in the hydrogenation reactor with a prescribed pressure in a regulator and generating a regulating parameter for the temperature via the deviation of the actual value from the prescribed value.

6. The method according to claim 1 or 2, wherein both the ratio of hydrogen reacted to amount of starting material fed in and the temperature in the hydrogenation reactor are kept constant.

7. The method according to any of claims 1 to 6, wherein the temperature in the hydrogenation reactor is set by means of the temperature of a heat transfer medium.

8. The method according to claim 7, wherein the heat transfer medium flows through at least one tube located in the hydrogenation reactor and/or through a reactor wall configured as a double wall.

9. The method according to any of claims 1 to 8, wherein the hydrogenation is carried out in the presence of a heterogeneous catalyst.

10. The method according to claim 9, wherein the catalyst comprises Cu, Pt, Rh, Pd or mixtures thereof as active components.

11. The method according to any of claims 1 to 10, wherein the hydrogenation reactor comprises at least one vapor phase and/or at least one liquid phase or a supercritical fluid.

12. The method according to any of claims 1 to 11, wherein the feed stream comprises one or more compounds selected from among maleic acid, maleic anhydride, maleic (mono)esters and further maleic anhydride derivatives, and one or more of the compounds selected from succinic acid, succinic anhydride, γ-butyrolactone, butanediol, tetrahydrofuran and butanol are obtained by the hydrogenation.

13. The method according to any of claims 1 to 12, wherein the hydrogenation is carried out at a temperature in the range from 170 to 300°C and at a pressure in the range from 5 to 100 bar.

## Revendications

1. Procédé pour contrôler une hydrogénation d'un produit de départ dans un réacteur d'hydrogénation, comprenant les étapes suivantes :
a) détermination de la quantité d'hydrogène transformée lors de l'hydrogénation,
b) détermination du rapport de la quantité d'hydrogène transformée à la quantité de produit de départ alimentée,
c) comparaison du quotient formé dans l'étape b) avec une valeur de consigne prédéfinie et
d) modification d'au moins un paramètre de procédé lorsque le quotient formé dans l'étape b) s'écarte d'une valeur prédéfinie de la valeur de consigne prédéfinie.

2. Procédé selon la revendication 1, **caractérisé en ce que** pour la détermination de la quantité d'hydrogène transformée, on forme la différence de la quantité d'hydrogène évacuée du processus et de la quantité d'hydrogène alimentée dans le processus.

3. Procédé selon la revendication 1 ou 2, **caractérisé en ce qu'**on génère, à partir de la comparaison réalisée dans l'étape c), une valeur de consigne pour un régulateur de température, qui règle la température dans le réacteur d'hydrogénation.

4. Procédé selon la revendication 3, **caractérisé en ce que** la pression dans le réacteur d'hydrogénation est maintenue constante au moyen d'une régulation de pression côté alimentation ou côté évacuation.

5. Procédé selon la revendication 1 ou 2, **caractérisé en ce que** le rapport de l'hydrogène transformé à la quantité alimentée de produit de départ est maintenu constant par le réglage de la quantité alimentée d'hydrogène et la pression qui se règle dans le réacteur d'hydrogénation est maintenue constante par le réglage de la température, **en ce que** la pression qui se règle dans le réacteur d'hydrogénation est comparée dans un régulateur avec une pression de consigne et une grandeur de régulation pour la température est générée par la déviation de la valeur instantanée de la valeur de consigne.

6. Procédé selon la revendication 1 ou 2, **caractérisé en ce que** le rapport d'hydrogène transformé à la quantité alimentée de produit de départ ainsi que la température dans le réacteur d'hydrogénation sont maintenus constants.

7. Procédé selon l'une quelconque des revendications 1 à 6, **caractérisé en ce que** la température dans le réacteur d'hydrogénation est réglée via la température d'un agent caloporteur.

8. Procédé selon la revendication 7, **caractérisé en ce que** l'agent caloporteur s'écoule dans au moins un tuyau disposé dans le réacteur d'hydrogénation et/ou dans une paroi de réacteur réalisée sous forme de double enveloppe.

9. Procédé selon l'une quelconque des revendications 1 à 8, **caractérisé en ce que** l'hydrogénation est réalisée en présence d'un catalyseur hétérogène.

10. Procédé selon la revendication 9, **caractérisé en ce que** le catalyseur contient, comme composant actif, Cu, Pt, Rh, Pd ou des mélanges de ceux-ci.

11. Procédé selon l'une quelconque des revendications 1 à 10, **caractérisé en ce que** le réacteur d'hydrogénation contient au moins une phase vapeur et/ou au moins une phase liquide ou un fluide supercritique.

12. Procédé selon l'une quelconque des revendications 1 à 11, **caractérisé en ce que** le flux de produits de départ contient un ou plusieurs des composés choisi(s) parmi l'acide maléique, l'anhydride de l'acide maléique, les (semi-)esters de l'acide maléique et d'autres dérivés de l'anhydride de l'acide maléique et qu'on obtient, par l'hydrogénation, un ou plusieurs des composés choisi(s) parmi l'acide succinique, l'anhydride de l'acide succinique, la γ-butyrolactone, le butanediol, le tétrahydrofuranne et le butanol.

13. Procédé selon l'une quelconque des revendications 1 à 12, **caractérisé en ce que** l'hydrogénation est réalisée à une température dans la plage de 170°C à 300°C et à une pression dans la plage de 5 bars à 100 bars.
